# EUROPEAN PATENT SPECIFICATION

(11) **EP 1 667 624 B1**
(45) Date of publication and mention of the grant of the patent: **13.06.2007**
(21) Application number: 04779147.0
(22) Date of filing: 27.07.2004
(51) Int. Cl.: A61F 13/58, A61F 13/15

(54) **CLOSURE SYSTEM AND METHOD OF MANUFACTURE**
VERSCHLUSSSYSTEM UND HERSTELLUNGSVERFAHREN
SYSTEME DE FERMETURE ET PROCEDE DE FABRICATION

(30) Priority: 29.09.2003 US 674174
(43) Date of publication of application: 14.06.2006
(73) Proprietor: 3M Innovative Properties Company, St. Paul, MN 55133-3427 (US)
(72) Inventor: WOOD, Leigh E., Saint Paul, MN 55133-3427 (US); JACKSON, Byron M., Saint Paul, MN 55133-3427 (US)
(74) Representative: Hermann, Gerhard
(86) International application number: PCT/US2004/023943
(87) International publication number: WO 2005/034825

(56) References cited:
- EP-A- 0 669 121
- EP-A- 0 793 953
- US-A- 5 599 601
- US-B1- 6 363 587

## Description

The manufacture of closure systems for use in articles such as, e.g., disposable diapers, may often involve adhesive attachment of the different components in the closure systems. Application of adhesives to the various components can, however raise issues with respect to control over delivery and placement of the adhesive. Problems with accurate adhesive delivery and placement can limit throughput in production lines. Adhesive control and placement can also adversely affect product quality if, e.g., the adhesive is misapplied or migrates to unwanted locations after application.

Many closure systems incorporate elastic materials to provide elasticity to the articles into which they are incorporated. If, e.g., the articles are garments designed to be worn, the elasticity of a closure system may be useful for providing dynamic fit or comfort as the wearer moves. In articles that are not designed to be worn by an individual, the elasticity may provide improved robustness to the closure system in response to forces that could otherwise cause the closure system to release undesirably.

Although elasticity may be a desirable characteristic, it can be difficult to incorporate elastic components into a closure system while still retaining the ability of the closure system to function properly. For example, it is generally difficult to provide mechanical fasteners, e.g., hook and loop closures, hermaphroditic closures, etc. on elastic substrates.

### SUMMARY OF THE INVENTION

The present invention provides a closure system that combines a base tab with a stable carrier tab for supporting a fastening component. The carrier tab overlaps only one side of the base tab and is connected to the base tab by a bonding tape. The bonding tape is located over the inner edge of the carrier tab with a portion of the bonding tape being attached to the base tab and another portion of the bonding tape being attached to the carrier tab. As a result, a portion of the carrier tab is located between the bonding tape and the base tab as disclosed in the US-A-5,599,601 patent.

Potential advantages of this construction are numerous. For example, the bonding tape may include a layer of adhesive facing the base tab and the carrier tab. As a result, adhesive attachment of the carrier tab to the base tab during manufacturing can be accomplished without applying adhesive alone to either the base tab or the carrier tab.

Another potential advantage of using an adhesive bonding tape is that, if the closure is to be used in a garment such as, e.g., a diaper, the potential for exposure of any adhesive to the skin of a wearer can be reduced or eliminated. The adhesive is located only on the bonding tape which is attached to the other components of the closure, thus eliminating exposed adhesive on the closure (unless that adhesive is used in the controlled environment of the fastener component as discussed herein).

The US-A-5,599,601 provides a closure system including a base tab with an outer edge and first and second major surfaces; a carrier tab having first and second major surfaces, an inner edge, and an opposing outer edge, the inner edge and the outer edge defining a length of the carrier tab; a fastener component attached to at least one of the first and second major surfaces of the carrier tab; an overlap region in which a portion of the first major surface of the carrier tab faces the second major surface of the base tab such that the outer edge of the base tab is located between the inner and outer edges of the carrier tab; and bonding tape attached to the second major surface of the base tab adjacent the overlap region, the bonding tape further attached to the first major surface of the carrier tab within the overlap region, wherein the inner edge of the carrier tab is located between the bonding tape and the second major surface of the base tab.

In one aspect, the present invention provides a closure system including an elastic base tab with an outer edge and first and second major surfaces; a carrier tab with first and second major surfaces, an inner edge, and an opposing outer edge, the inner edge and the outer edge defining a length of the carrier tab, wherein the carrier tab is inelastic; a fastener component attached to at least one of the first and second major surfaces of the carrier tab; an overlap region in which a portion of the first major surface of the carrier tab faces the second major surface of the elastic base tab such that the outer edge of the elastic base tab is located between the inner and outer edges of the carrier tab; and a bonding tape adhesively attached and welded to the second major surface of the elastic base tab adjacent the overlap region, the bonding tape further adhesively attached and welded to the first major surface of the carrier tab within the overlap region, wherein the inner edge of the carrier tab is located between the bonding tape and the second major surface of the elastic base tab, and further wherein the bonding tape is inelastic.

The US-A,5,599,601 provides a method of manufacturing a composite web for closure systems by providing a base tab web having an outer edge and first and second major surfaces; providing a carrier tab web having first and second major surfaces, an inner edge, and an opposing outer edge; aligning the base tab web and the carrier tab web to form an overlap region in which a portion of the first major surface of the carrier tab web faces the second major surface of the base tab web such that the outer edge of the base tab web is located between the inner and outer edges of the carrier tab web; aligning a bonding tape over the inner edge of the carrier tab web, wherein the inner edge of the carrier tab web is located between the bonding tape and the second major surface of the base tab web; attaching the bonding tape to the second major surface of the base tab web adjacent the overlap region; attaching the bonding tape to the first major surface of the carrier tab web within the overlap region; and attaching a fastener component web to at least one of the first and second major surfaces of the carrier tab web.

In another aspect, the present invention provides a method of manufacturing a composite web for closure systems by providing an elastic base tab web having an outer edge and first and second major surfaces; providing a carrier tab web having first and second major surfaces, an inner edge, and an opposing outer edge, wherein the carrier tab web is inelastic; aligning the elastic base tab web and the carrier tab web to form an overlap region in which a portion of the first major surface of the carrier tab web faces the second major surface of the elastic base tab web such that the outer edge of the elastic base tab web is located between the inner and outer edges of the carrier tab web; aligning a bonding tape over the inner edge of the carrier tab web, wherein the inner edge of the carrier tab web is located between the bonding tape and the second major surface of the elastic base tab web, and wherein the bonding tape is inelastic, and further wherein the bonding tape includes a layer of pressure sensitive adhesive facing the elastic base tab web and the carrier tab web; adhesively attaching and welding the bonding tape to the second major surface of the elastic base tab web adjacent the overlap region; adhesively attaching and welding the bonding tape to the first major surface of the carrier tab web within the overlap region; and attaching a fastener component web to at least one of the first and second major surfaces of the carrier tab web.

These and other features and advantages of the present invention may be described in more detail below with respect to various illustrative embodiments of the invention.

### BRIEF DESCRIPTION OF THE DRAWINGS

FIG. 1 is an edge view of one closure system according to the present invention.
FIG. 2 is a plan view of the closure system of FIG. 1 taken from below the closure system as seen in FIG. 1.
FIG. 3 is a diagram of one process of manufacturing a closure system according to the present invention.
FIG. 4 is a view of two closure systems of the present invention attached to an article.

### DETAILED DESCRIPTION OF ILLUSTRATIVE EMBODIMENTS OF THE INVENTION

In the following detailed description of illustrative embodiments, reference is made to the accompanying figures of the drawing which form a part hereof, and in which are shown by way of illustration specific embodiments in which the invention may be practiced. It is to be understood that other embodiments may be utilized and structural changes may be made without departing from the scope of the present invention.

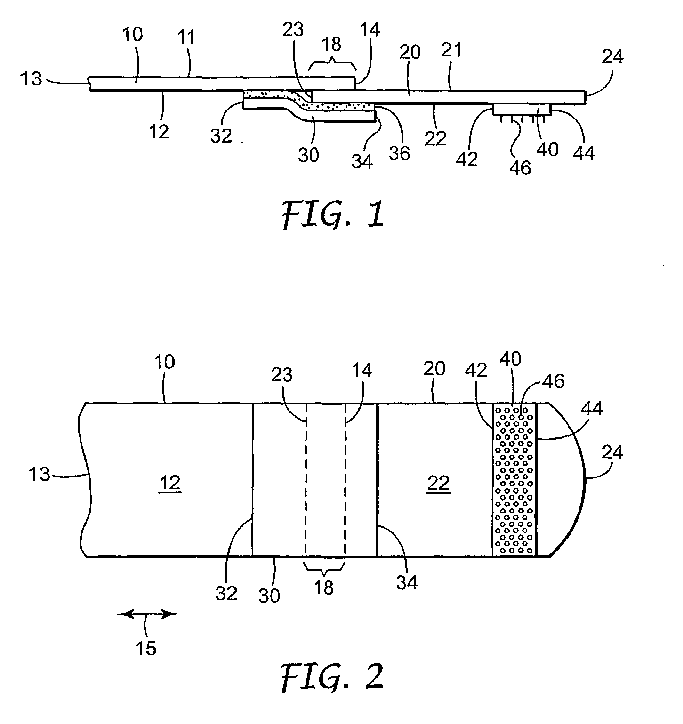

FIG. 1 is an edge view and FIG. 2 is a plan view of one illustrative embodiment of a closure system according to the present invention. The closure system includes a base tab 10, a carrier tab 20, a bonding tape 30 and a fastener component 40 located on the carrier tab 20. The view of FIG. 2 is taken from below the edge view depicted in FIG. 1, i.e., from the side on which the bonding tape 30 is located.

The base tab 10 includes first and second major surfaces 11 and 12 (respectively). The base tab 10 also includes an inner edge 13 and an outer edge 14. It may be preferred that the base tab 10 be flexible such that it can be processed using conventional web processing equipment.

The base tab 10 may exhibit elasticity in primarily the directions indicated by double-headed arrow 15 in FIG. 2, or it may exhibit elasticity in any or all directions. Elasticity in all directions is not, however required for an elastic base tab 10. As used herein "elastic" and "elasticity" (and variations thereof) refer to the ability of the base tab 10 or at least a portion of the tab, if elongated in response to a load, to recover to a state that is at or near it's original state (i.e., before elongation) after removal of the load causing the elongation. The recovery preferably occurs within a relatively short period of time (e.g., one minute or less).

Suitable sheet materials for the base tab 10 may be unitary, homogeneous materials (e.g., polymeric films, nonwovens, woven fabrics, knitted fabrics, paper, etc.). Alternatively, they may be laminated or composite sheet materials. If the base tab 10 is to be elastic or include portions that are elastic, laminated sheet materials used for the base tab 10 may include only one or more components that exhibits elasticity, but in which the laminated sheet material as a whole exhibits desired elastic characteristics.

Examples of some potentially suitable elastic sheet materials for the base tab 10 may be described in, e.g., U.S. Patent Nos. 5,653,704 (Buell et al.); 5,807,368 (Helmer); 5,840,412 (Wood et al.); 5,885,908 (Jaeger et al.); 6,159,584 (Eaton et al.); and 6,270,910 (Jaeger et al.).

Elastic sheet materials suitable for the base tab 10 may or may not be activated (i.e., stretched to activate the elastic properties of the sheet material as is known in the art). The sheet materials used for the base tabs 10 may include portions or zones that do not exhibit elasticity (either because they are incapable of exhibiting elasticity or they have not been activated). Also, the sheet materials used for the base tab 10 may include one or more nonwoven fibrous outer layers (as described in, e.g., U.S. Patent No. 5,807,368 to Helmer).

Although the base tab 10 is depicted as having an inner edge 13, it should be understood that in some instances, the base tab 10 will be an integral extension or portion of a larger article (e.g., a diaper, adult incontinence device, gown, etc) and, as such, may not have a defined inner edge 13 as depicted in connection with the illustrative closure system of FIGS. 1 & 2.

The outer edge 14 of the base tab 10 is the edge from which the carrier tab 20 extends when attached to the closure system. Although the outer edge 14 is depicted as a straight edge, it will be understood that outer edge 14 could take any suitable shape, e.g., curvilinear, sinusoidal, sawtooth, etc.

The carrier tab 20 includes first and second major surfaces 21 and 22 along with an inner edge 23 and an outer edge 24. The carrier tab 20 may be manufactured of any suitable sheet material, but may preferably be inelastic. As used herein, "inelastic" means that the carrier tab 20 will not exhibit significant recovery after elongation in response to a load, i.e., substantially all of the elongation will be permanent. It may be preferred that, in addition to preferably being inelastic, the carrier tab 20 be substantially inextensible when used as intended in an article (e.g., a disposable garment, diaper, incontinence device, etc.).

Suitable materials for the carrier tab 20 may be any sheet material that provides the desired inelastic and inextensible characteristics. It may be preferred that the carrier tab 20 be flexible such that it can be processed using conventional web processing equipment. Examples of suitable materials may include unitary, homogeneous materials (e.g., films, nonwovens, woven fabrics, etc.). Alternatively, the suitable materials may be laminated or composite sheet structures that, as a whole, exhibit the desired characteristics, e.g., inelasticity, inextensibility, etc. Regardless of the specific constructions of the materials for the carrier tab 20, the materials preferably provide at least an area of at least one surface suitable for attachment of a fastener component as discussed herein.

Examples of some potentially suitable sheet materials for the carrier tab are cloth (woven, knitted, etc.), paper, cellophane film, polymeric films (e.g., polyolefin, poly (ethylene terephthalate), poly(vinyl chloride), etc.), nonwoven materials (such as, e.g., meltblown or spunbond webs), etc. Laminates may also be used as sheet materials for the carrier tab, e.g., polymeric film/nonwoven laminates, etc. Suitable polymeric sheet materials for the carrier tab 20 may be described in, e.g., U.S. Patent Nos. 4, 237,889 (Gobran); 4,769,283 (Sipinen et al.); and 4,808,474 (Sipinen).

The base tab 10 and the carrier tab 20 are attached to each other using the bonding tape 30. Bonding tape 30 is attached to major surface 12 of the base tab 10 and major surface 22 of the carrier tab 20. As such, the iimer edge 23 of the carrier tape 20 is located between major surface 12 of base tab 10 and the bonding tape 30. Bonding tape 30 includes an inner edge 32 and an outer edge 34, with the inner edge 32 being located on major surface 12 of the base tab 10 between inner edge 13 and outer edge 14 of the base tab 10. The outer edge 34 of the bonding tape 30 is located on second major surface 22 of the carrier tab 20 between the inner edge 23 and the outer edge 24 of the carrier tab 20.

Bonding tape 30 is preferably inelastic. As a result, the elasticity of the portion of the base tab 10 attached to the bonding tape 30 is restrained, i.e., it does not operate elastically because it is constrained by the inelastic nature of the bonding tape 30.

Bonding tape 30 may preferably, but not necessarily, include a layer of adhesive 36 on its surface facing the base tab 10 and the carrier tab 20. The adhesive 36 may preferably be a pressure-sensitive adhesive such as the pressure- sensitive adhesives described in, e.g., U.S. Patent Nos. 3,932,328 (Korpman) and 5,019,071 (Bany et al.). Other attachment or bonding techniques may be used in place of pressure sensitive adhesives, e.g., heat-activated bonding (as described in, e.g., U.S. Patent No. 4,973,326 (Wood et al.)) or the construction adhesives described in connection with disposable diaper manufacturing in U.S. Patent Nos. 5,149,741 (Alper et al.) and 6,184,285 B1 (Hatfield et al.).

In some instances, the only attachment between the bonding tape 30 and the base tab 10 and carrier tab 20 may be an adhesive attachment using the adhesive 36 on the bonding tape 30. Alternatively, it may be preferred to supplement the adhesive attachment provided by the adhesive 36 by welding the bonding tape 30 to the base tab 10 and/or the carrier tab 20. The welding may be performed by any suitable technique, e.g., chemical welds (using, e.g., solvents), ultrasonic welds, heat sealing, heat welding, etc. Another welding technique that may be used is dynamic mechanical welding as described in, e.g., U.S. Patent Nos. 4,854,984 and 4,919,738 (both to Ball et al.).

If welding is to be used as a supplement to adhesive attachment, the adhesive 36 should be compatible with the welding technique to be employed, both in the composition of the adhesive 36 and in how it is provided on the bonding tape 30. For example, it may be desirable to provide a thinner layer of adhesive 36 on the bonding tape 30 if welding is to be used to supplement the adhesive. The thinner layer of adhesive may preferably provide a positioning bond to position the base tab 10, carrier tab 20 and bonding tape 30 in the manufacturing process until the bonding tape 30 can be welded into place. Other alternatives to a thinner layer of adhesive may include pattern coating the bonding tape 30 such that areas to be welded may be free of or include thinner layers of adhesive 36 than the surrounding areas on the bonding tape.

Also, if welding is to be used to attach the bonding tape 30 to the base tab 10 and the carrier tab 20, the materials present on the surfaces facing each other are preferably compatible with the welding technique to be used. For example, the bonding tape 30 may preferably have a film backing amenable to welding or be a composite that includes a film layer that is amenable to welding. Similarly, the second major surface 12 of the base tab 10 and the second major surface 22 of the carrier tab 20 to which the bonding tape 30 is welded are also preferably of a composition that is amenable to welding.

Although the bonding tape 30 is depicted with adhesive 36, the use of an adhesive to attach the bonding tape 30 to the base tab 10 and the carrier tab 20 is optional. In some instances the bonding tape 30 may be attached to the base tab 10 and/or carrier tab solely by welding. The welding may be performed by any suitable technique, e.g., chemical welds (using, e.g., solvents), ultrasonic welds, heat sealing, heat welding, etc. Another welding technique that may be used is dynamic mechanical welding as described in, e.g., U.S. Patent Nos. 4,854,984 and 4,919,738 (both to Ball et al.).

In the arrangement of the base tab 10 and the carrier tab 20, it may be preferred that the two tabs overlap each other such that an overlap region 18 be created. The boundaries of the overlap region 18 are defined by the outer edge 14 of the base tab 10 and the inner edge 23 of the carrier tab 20 (along with the sides of the base tab 10 and the carrier tab 20). As a result, the outer edge 14 of the base tab 10 is located between the inner edge 23 and the outer edge 24 of the carrier tab 20. The boundaries of the overlap region 18 are seen in FIG. 2 where the outer edge 14 of the base tab 10 and the inner edge 23 of the carrier tab 20 are depicted as hidden (broken) lines because they are located on the opposite side of the bonding tape 30.

Within the overlap region 18, the major surface 21 of the carrier tab 20 faces the major surface 12 of the base tab 10. In the depicted embodiment, no adhesive is provided between the major surface 21 of the carrier tab 20 and the major surface 12 of the base tab 10, i.e., the surface 21 of the carrier tab 20 is not adhered to the surface 12 of the base tab 10. Rather, the attachment of the base tab 10 and the carrier tab 20 is accomplished through the bonding tape 30 which is attached to the second major surface 12 of the base tab 10 and the second major surface 22 of the carrier tab 20.

The overlap region 18 may preferably be located within the boundaries of the bonding tape 30. By overlapping the base tab 10 and the carrier tab 20, any adhesive 36 that is on the side of the bonding tape 30 facing the base tab 10 and the carrier tab 20 is covered by either or both of the base tab 10 and the carrier tab 20. Alternatively, the overlap region 18 may extend outside of the boundaries of the boding tape 30. For example, the outer edge 14 of the base tab 10 may be located between the outer edge 34 of the bonding tape 30 and the outer edge 24 of the carrier tab 20. In the depicted embodiment, however, the outer edge 14 of the base tab 10 is located between the inner edge 32 and the outer edge 34 of the bonding tape 30, i.e., within the boundaries of the bonding tape 30.

The bonding tape 30 in the illustrative embodiment of FIGS. 1 & 2 also depicts another optional feature, i.e., that the bonding tape 30 is coextensive with the width of the carrier tab 20, where the width of the carrier tab 20 is measured transverse to the direction defined by double-headed arrow 15 in FIG. 2. Although the bonding tape 30 is also coextensive with the width of the base tab 10, the bonding tape 30 may not always be coextensive with the base tab 10 (when, e.g., the carrier tab 20 has a narrower width than the base tab 10).

The closure system of FIGS. 1 & 2 also includes a fastener component 40 located on the carrier tab 20. The fastener component 40 may be attached to the carrier tab 20 by any suitable technique or combination of techniques. Some suitable techniques may include one or more adhesives, welding (thermal, ultrasonic, dynamic mechanical bonding, chemical, etc.), clips, staples, sewing, etc.

Although the depicted fastener component 40 is attached to the second major surface 22 of the carrier tab 20, it could alternatively be attached to the first major surface 21. In still another alternative, fastener components could be attached to both the first and second major surfaces 21 and 22 of the carrier tab 20.

The depicted fastener component 40 is a hook mechanical fastener that includes stems 46 (e.g., hooks, mushroom-shaped structures, etc.) to fasten articles together. Such fastening systems are well known in the art and some suitable examples may be described in U.S. Patent Nos. 4,894,060 (Nestegard) and 5,077,870 (Melbye et al.). The fastener component 40 may alternatively be the loop component of a hook-and-loop closure system. Some loop components are described in, e.g., U.S. Patent Nos. 5,616,394 (Gorman) and 5,605,729 (Mody). In place of hook-and-loop closures, the fastener component 40 may rely on any known fastening technology, e.g., pressure-sensitive adhesive, hermaphroditic closures, cohesive materials, non-tacky adhesive, etc.

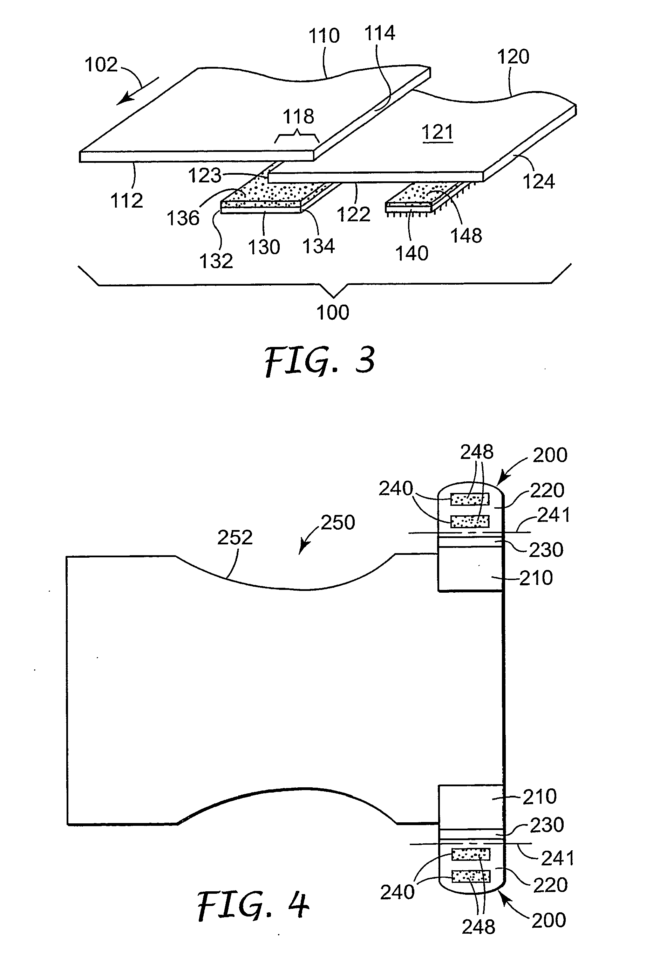

Turning to FIG. 3, a diagram of one method of manufacturing closure systems is depicted. The manufacturing method involves the use of webs for each of the components in the closure system such that high speed and/or economical manufacturing can be provided. The webs provided to manufacture the closure systems include a base tab web 110, a carrier tab web 120, a bonding tape 130, and a fastener web 140 which, taken together, form a composite web 100. The webs and resulting composite web 100 preferably progress along a machine direction as indicated by arrow 102 through web processing equipment during the manufacturing process.

The base tab web 110 may be an elastic sheet material of which all of the web 110 is elastic. Alternatively, the sheet materials used for the base tab web 110 may include one or more portions or zones that do not exhibit elasticity (either because they are incapable of exhibiting elasticity or they have not been activated).

As seen in FIG. 3, the base tab web 110 and the carrier tab web 120 are aligned such that they form an overlap region 118 in which a portion of the major surface 121 of the carrier tab web 120 faces the major surface 112 of the base tab web 110. Because of the overlap region 118, the outer edge 114 of the base tab web 110 is located between the inner edge 123 and the outer edge 124 of the carrier tab web 120. It is preferred that no adhesive be located between the major surface 121 of the carrier tab web 120 and the major surface 112 of the base tab web 110 within the overlap region 118.

The depicted method also includes aligning the bonding tape 130 over the inner edge 123 of the carrier tab web 120 such that the inner edge 123 of the carrier tab web 120 is located between the bonding tape 130 and major surface 112 of the base tab web 110.

The bonding tape 130 is attached to the major surface 112 of the base tab web 110 adjacent the overlap region 118, i.e., between the edge 132 of the bonding tape 130 and the inner edge 123 of the carrier tab web 120. The bonding tape 130 is also attached to major surface 122 of the carrier tab web 120, i.e., between the inner edge 123 of the carrier tab web 120 and edge 134 of the bonding tape 130.

The bonding tape 130 may be attached to the base tab web 110 and the carrier tab web 120 by any suitable technique or combination of techniques. Various approaches to attaching the bonding tape 130 to the base tabs and carrier tabs are described above in connection with FIGS. 1 & 2 and that discussion will not be repeated here except to note that the attachment of the various webs may occur in any desired order. For example, the bonding tape 130 may be attached to the carrier tab web 120 before or after the base tab web 110 is aligned with the carrier tab web 120 to define the overlap region 118. Alternatively, alignment of the base tab web 110 and the carrier tab web 120 may occur before the bonding tape is attached to either web.

If an adhesive 136 is provided on the bonding tape 130 as seen in FIG. 3, the adhesive 136 may preferably be applied to the bonding tape 130 in an off-line operation. For example, the adhesive 136 may preferably be coated on or transferred to the bonding tape 130 before the bonding tape 130 separately, followed by winding of the bonding tape 130 with the adhesive 136. When used in the depicted method, the bonding tape 130 with adhesive 136 is unwound and directed into the process. Those skilled in the art of web processing will, however, understand that the adhesive 136 could alternatively be coated with adhesive in-line with the manufacturing process depicted in FIG. 3.

Another portion of the method depicted in FIG. 3 is the alignment and attachment of the fastener component web 140 to the carrier tab web 120. In the depicted method, the fastener component web 140 includes an adhesive layer 148 that may be used to attach the fastener component web 140 to major surface 122 of the carrier tab web 120, although any suitable technique or combination of techniques could be used to attach the fastener component web 140 to the carrier tab web 120.

Also, although the fastener component web 140 is depicted as being attached to the major surface 122 of carrier tab web 120, it will be understood that the fastener component web 140 could be attached to the opposite major surface 121 of the carrier tab web 120. In another alternative, fastener component webs could be attached to both major surfaces 121 and 122 of carrier tab web 120. It is also contemplated that the fastener components, if attached to both major surfaces 121 and 122, may not be the same. As one example, the fastener component on one major surface may be a hook fastener while the fastener component on the opposite major surface may the complementary loop fastener. Also, more than one fastener component web could be attached to any one of the major surfaces 121 and 122 of the carrier tab web 120.

After the various webs have been aligned and attached, closure systems (such as those depicted in FIGS. 1 & 2) can be produced by separating the composite web 100 along a direction generally transverse to the machine direction as indicated by arrow 102. Furthermore, although the composite web 100 depicted in FIG. 3 can be sheeted into a single or "one-up" row of closure systems, it will be understood that carrier tab web 120 could be widened and an additional base tab web, bonding tape 130 and fastener component web 140 could be attached to form a "two-up" composite web that could be sheeted into two rows of closure systems. Example of sheeting patterns that may be used to separate "two-up" composite webs are depicted in, e.g., FIG. 4 of U.S. Patent No. 5,399,219 (Roessler et al.) and FIG. 9B of U.S. Patent No. 6,406,467 B1 (Dilnik et al.).

FIG. 4 is a plan view of an article that includes a pair of closure systems manufactured in accordance with the present invention. The depicted article 250 is an absorbent garment, e.g., a diaper or adult incontinence device. As discussed above, however, the closure systems of the present invention may be used on any garment (e.g., a gown, robe, vest, etc.) or any other article requiring a fastening element (e.g., cable ties, bedding, etc.)

The article 250 includes a pair of closure systems 200 attached to a chassis 252. The base tabs 210 of each closure system 200 are preferably elastic as discussed above and may be attached to the chassis by any suitable technique or combination of techniques. Suitable techniques may include one or more adhesives, welding (thermal, ultrasonic, dynamic mechanical bonding, chemical, etc.), clips, staples, sewing, etc.

The closure systems 200 each include a pair of fastener components 240 on a carrier tab 220 that is attached to the base tab 210 by a bonding tape 230. Each fastener component 240 includes an exposed pressure-sensitive adhesive 248 that, when attached to the backside of the chassis 252, retains the article in place on a wearer (where the backside of the chassis is the side facing away from a viewer of FIG. 4). It should be understood that a complementary fastener component may preferably be provided on the backside of the chassis 252 to provide for secure attachment of the fastener component 240 as described in, e.g., U.S. Patent Nos. 5,300,057 (Miller et al.) and 5,660,666 (Dilnik et al.).

In the depicted embodiment, the adhesive fastener components 240 are provided in the form of patches that do not extend over the width (along axis 241) of the closure systems 200. Alternatively, the fastener components may extend over the width of the closures systems 200 as seen in, e.g., FIG. 2.

Illustrative embodiments of this invention are discussed and reference has been made to possible variations within the scope of this invention. These and other variations and modifications in the invention will be apparent to those skilled in the art without departing from the scope of the invention, and it should be understood that this invention is not limited to the illustrative embodiments set forth herein. Accordingly, the invention is to be limited only by the claims provided below and equivalents thereof.

## Claims

1. A closure system comprising:
a base tab comprising an outer edge and first and second major surfaces;
a carrier tab comprising first and second major surfaces, an inner edge, and an opposing outer edge, the inner edge and the outer edge defining a length of the carrier tab;
a mechanical fastener component attached to at least one of the first and second major surfaces of the carrier tab;
an overlap region in which a portion of the first major surface of the carrier tab faces the second major surface of the base tab such that the outer edge of the base tab is located between the inner and outer edges of the carrier tab wherein no adhesive is located between the first major surface of the carrier tab and the second major surface of the base tab within the overlap region; and
bonding tape adhesively attached and welded to the second major surface of the base tab adjacent the overlap region, the bonding tape further adhesively attached and welded to the second major surface of the carrier tab within the overlap region, wherein the inner edge of the carrier tab is located between the bonding tape and the second major surface of the base tab.

2. A closure system according to claim 1, wherein the bonding tape comprises a layer of pressure sensitive adhesive facing the base tab and the carrier tab, wherein the pressure sensitive adhesive is compatible with the welding technique used to attach the bonding tape to the the base tab and the carrier tab.

3. A closure system according to any one of claims 1-2, wherein at least a portion of the base tab exhibits elasticity.

4. A closure system according to any one of claims 1-3, wherein the carrier tab is inelastic.

5. A closure system according to any one of claims.1-4, wherein the bonding tape is inelastic.

6. A closure system according to any.one of claims 1-5, wherein the base tab comprises an integral portion of a disposable garment.

7. A closure system according to any one of claims 1-6, wherein the fastener component is adhesively attached to the carrier tab.

8. A closure system according to any one of claims 1-7, wherein the bonding tape is coextensive with a width of the carrier tab as measured transverse to the length of the carrier tab.

9. A closure system according to any one of claims 1-8, wherein the fastener component is coextensive with a width of the carrier tab and the base tab as measured transverse to the length of the carrier tab.

10. A method of manufacturing a composite web for closure systems, the method comprising:
providing a base tab web comprising an outer edge and first and second major surfaces;
providing a carrier tab web comprising first and second major surfaces, an inner edge, and an opposing outer edge;
aligning the base tab web and the carrier tab web to form an overlap region in which a portion of the first major surface of the carrier tab web faces the second major surface of the base tab web such that the outer edge of the base tab web is located between the inner and outer edges of the carrier tab web, wherein no adhesive is located between the first major surface of the carrier tab web and the second major surface of the base tab web within the overlap region;
aligning a bonding tape over the inner edge of the carrier tab web, wherein the inner edge of the carrier tab web is located between the bonding tape and the second major surface of the base tab web;
adhesively attaching and welding the bonding tape to the second major surface of the base tab web adjacent the overlap region;
adhesively attaching and welding the bonding tape to the second major surface of the carrier tab web within the overlap region; and
attaching a mechanical fastener component web to at least one of the first and second major surfaces of the carrier tab web.

11. A method according to claim 10, wherein the bonding tape comprises a layer of pressure sensitive adhesive facing the base tab web and the carrier tab web, wherein the pressure sensitive adhesive is compatible with the welding technique used to attach the bonding tape to the base tab and the carrier tab.

12. A method according to any one of claims 10-11, wherein at least a portion of the base tab web comprises elastic sheet material.

13. A method according to any one of claims 10-12, wherein the carrier tab web is inelastic.

14. A method according to any one of claims 10-13, wherein the bonding tape is inelastic.

15. A method according to any one of claims 10-14, wherein attaching the fastener component web to the carrier tab web comprises adhesively attaching the fastener component web to the carrier tab web.

16. A method according to any one of claims 10-15, further comprising separating the composite web into a plurality of discrete closure systems after attaching the bonding tape web to the base tab web and the carrier tab web and attaching the fastener component web to the carrier tab web, wherein each discrete closure system of the plurality of discrete closure systems comprises a base tab, carrier tab, a bonding tape, and a fastener component of the same width.

## Patentansprüche

1. Verschlusssystem, aufweisend:
einen Basisstreifen, aufweisend einen Außenrand und eine erste und eine zweite Hauptfläche;
einen Trägerstreifen, aufweisend eine erste und eine zweite Hauptfläche, einen Innenrand und einen gegenüberliegenden Außenrand, wobei der Innenrand und der Außenrand eine Länge des Trägerstreifens definieren;
eine mechanische Befestigungskomponente, die an mindestens einer der ersten und zweiten Hauptflächen des Trägerstreifens angebracht ist;
einen Überlappungsbereich, in welchem ein Teil der ersten Hauptfläche des Trägerstreifens der zweiten Hauptfläche des Basisstreifens derart zugewandt ist, dass sich der Außenrand des Basisstreifens zwischen dem Innen- und dem Außenrand des Trägerstreifens befindet, wobei sich zwischen der ersten Hauptfläche des Trägerstreifens und der zweiten Hauptfläche des Basisstreifens innerhalb des Überlappungsbereichs kein Klebstoff befindet; und
Klebeband, das an die zweite Hauptfläche des Basisstreifens neben dem Überlappungsbereich klebend angebracht und angeschweißt ist, wobei das Klebeband ferner an die zweite Hauptfläche des Trägerstreifens innerhalb des Überlappungsbereichs klebend angebracht und angeschweißt ist, wobei
sich der Innenrand des Trägerstreifens zwischen dem Klebeband und der zweiten Hauptfläche des Basisstreifens befindet.

2. Verschlusssystem nach Anspruch 1, wobei das Klebeband eine druckempfindliche Klebstoffschicht aufweist, die dem Basisstreifen und dem Trägerstreifen zugewandt ist, wobei der druckempfindliche Klebstoff mit der zum Anbringen des Klebebands am Basisstreifen und am Trägerstreifen verwendeten Schweißtechnik verträglich ist.

3. Verschlusssystem nach einem der Ansprüche 1-2, wobei zumindest ein Teil des Basisstreifens Elastizität aufweist.

4. Verschlusssystem nach einem der Ansprüche 1-3, wobei der Trägerstreifen unelastisch ist.

5. Verschlusssystem nach einem der Ansprüche 1-4, wobei das Klebeband unelastisch ist.

6. Verschlusssystem nach einem der Ansprüche 1-5, wobei der Basisstreifen einen integralen Teil eines Einwegkleidungsstücks aufweist.

7. Verschlusssystem nach einem der Ansprüche 1-6, wobei die Befestigungskomponente am Trägerstreifen klebend angebracht ist.

8. Verschlusssystem nach einem der Ansprüche 1-7, wobei das Klebeband mit einer Breite des Trägerstreifens, gemessen quer zur Länge des Trägerstreifens, koextensiv ist.

9. Verschlusssystem nach einem der Ansprüche 1-8, wobei die Befestigungskomponente mit einer Breite des Trägerstreifens und des Basisstreifens, gemessen quer zur Länge des Trägerstreifens, koextensiv ist.

10. Verfahren zur Herstellung einer Verbundbahn für Verschlusssysteme, wobei das Verfahren aufweist:
Bereitstellen einer Basisstreifenbahn, aufweisend einen Außenrand und eine erste und eine zweite Hauptfläche;
Bereitstellen einer Trägerstreifenbahn, aufweisend eine erste und eine zweite Hauptfläche, einen Innenrand und einen gegenüberliegenden Außenrand;
Ausrichten der Basisstreifenbahn und der Trägerstreifenbahn zur Bildung eines Überlappungsbereichs, in welchem ein Teil der ersten Hauptfläche der Trägerstreifenbahn der zweiten Hauptfläche der Basisstreifenbahn zugewandt ist, so dass sich der Außenrand der Basisstreifenbahn zwischen dem Innen- und dem Außenrand der Trägerstreifenbahn befindet, wobei sich zwischen der ersten Hauptfläche der Trägerstreifenbahn und der zweiten Hauptfläche der Basisstreifenbahn innerhalb des Überlappungsbereichs kein Klebstoff befindet;
Ausrichten eines Klebebands über dem Innenrand der Trägerstreifenbahn, wobei sich der Innenrand der Trägerstreifenbahn zwischen dem Klebeband und der zweiten Hauptfläche der Basisstreifenbahn befindet;
klebendes Anbringen und Anschweißen des Klebebands an die zweite Hauptfläche der Basisstreifenbahn neben dem Überlappungsbereich;
klebendes Anbringen und Anschweißen des Klebebands an die zweite Hauptfläche der Trägerstreifenbahn innerhalb des Überlappungsbereichs; und
Anbringen einer mechanischen Befestigungskomponentenbahn an mindestens eine der ersten und zweiten Hauptflächen der Trägerstreifenbahn.

11. Verfahren nach Anspruch 10, wobei das Klebeband eine druckempfindliche Klebstoffschicht aufweist, die der Basisstreifenbahn und der Trägerstreifenbahn zugewandt ist, wobei der Klebstoff mit der zum Anbringen des Klebebands am Basisstreifen und am Trägerstreifen verwendeten Schweißtechnik verträglich ist.

12. Verfahren nach einem der Ansprüche 10-11, wobei zumindest ein Teil der Basisstreifenbahn elastisches Lagenmaterial aufweist.

13. Verfahren nach einem der Ansprüche 10-12, wobei die Trägerstreifenbahn unelastisch ist.

14. Verfahren nach einem der Ansprüche 10-13, wobei das Klebeband unelastisch ist.

15. Verfahren nach einem der Ansprüche 10-14, wobei das Anbringen der Befestigungskomponentenbahn an die Trägerstreifenbahn das klebende Anbringen der Befestigungskomponentenbahn an die Trägerstreifenbahn aufweist.

16. Verfahren nach einem der Ansprüche 10-15, ferner aufweisend das Auftrennen der Verbundbahn in mehrere direkte Verschlusssysteme nach Anbringen der Klebebandbahn an die Basisstreifenbahn und die Trägerstreifenbahn und Anbringen der Befestigungskomponentenbahn an die Trägerstreifenbahn, wobei jedes diskrete Verschlusssystem der mehreren diskreten Verschlusssysteme einen Basisstreifen, einen Trägerstreifen, ein Klebeband und eine Befestigungskomponente derselben Breite aufweist.

## Revendications

1. Système de fermeture, comprenant:
un onglet de base présentant un bord extérieur et des première et deuxième surfaces principales;
un onglet de support présentant des première et deuxième surfaces principales, un bord intérieur et un bord extérieur opposé, le bord intérieur et le bord extérieur définissant une longueur de l'onglet de support;
un composant de fixation mécanique fixé à au moins une des première et deuxième surfaces principales de l'onglet de support;
une région de recouvrement dans laquelle une partie de la première surface principale de l'onglet de support fait face à la deuxième surface principale de l'onglet de base de telle sorte que le bord extérieur de l'onglet de base soit situé entre les bords intérieur et extérieur de l'onglet de support, aucun adhésif n'étant placé entre la première surface principale de l'onglet de support et la deuxième surface principale de l'onglet de base à l'intérieur de la région de recouvrement; et
un ruban de liaison fixé de façon adhésive et soudé à la deuxième surface principale de l'onglet de base à proximité de la région de recouvrement, le ruban de liaison étant en outre fixé de façon adhésive et soudé à la deuxième surface principale de l'onglet de support à l'intérieur de la région de recouvrement, le bord intérieur de l'onglet de support étant situé entre le ruban de liaison et la deuxième surface principale de l'onglet de base.

2. Système de fermeture selon la revendication 1, dans lequel le ruban de liaison comprend une couche d'un adhésif sensible à la pression faisant face à l'onglet de base et à l'onglet de support, dans lequel l'adhésif sensible à la pression est compatible avec la technique de soudage utilisée pour fixer le ruban de liaison à l'onglet de base et à l'onglet de support.

3. Système de fermeture selon l'une quelconque des revendications 1 et 2, dans lequel au moins une partie de l'onglet de base présente une certaine élasticité.

4. Système de fermeture selon l'une quelconque des revendications 1 à 3, dans lequel l'onglet de support est inélastique.

5. Système de fermeture selon l'une quelconque des revendications 1 à 4, dans lequel le ruban de liaison est inélastique.

6. Système de fermeture selon l'une quelconque des revendications 1 à 5, dans lequel l'onglet de base comprend une partie intégrée d'un vêtement jetable.

7. Système de fermeture selon l'une quelconque des revendications 1 à 6, dans lequel le composant de fixation est fixé de façon adhésive à l'onglet de support.

8. Système de fermeture selon l'une quelconque des revendications 1 à 7, dans lequel le ruban de liaison s'étend sur la même largeur que l'onglet de support, mesurée transversalement à la longueur de l'onglet de support.

9. Système de fermeture selon l'une quelconque des revendications 1 à 8, dans lequel le composant de fixation s'étend sur la même largeur que l'onglet de support et l'onglet de base, mesurée transversalement à la longueur de l'onglet de support.

10. Procédé de fabrication d'une bande composite pour des systèmes de fermeture, le procédé comprenant:
la fourniture d'une bande d'onglet de base présentant un bord extérieur et des première et deuxième surfaces principales;
la fourniture d'une bande d'onglet de support présentant des première et deuxième surfaces principales, un bord intérieur et un bord extérieur opposé;
l'alignement de la bande d'onglet de base et de la bande d'onglet de support pour former une région de recouvrement dans laquelle une partie de la première surface principale de la bande d'onglet de support fait face à la deuxième surface principale de la bande d'onglet de base de telle sorte que le bord extérieur de la bande d'onglet de base soit situé entre les bords intérieur et extérieur de la bande d'onglet de support, aucun adhésif n'étant placé entre la première surface principale de la bande d'onglet de support et la deuxième surface principale de la bande d'onglet de base à l'intérieur de la région de recouvrement;
l'alignement d'un ruban de liaison sur le bord intérieur de la bande d'onglet de support, le bord intérieur de la bande d'onglet de support étant situé entre le ruban de liaison et la deuxième surface principale de la bande d'onglet de base;
la fixation adhésive et le soudage du ruban de liaison à la deuxième surface principale de la bande d'onglet de base à proximité de la région de recouvrement;
la fixation adhésive et le soudage du ruban de liaison à la deuxième surface principale de la bande d'onglet de support à l'intérieur de la région de recouvrement; et
la fixation d'une bande de composant de fixation mécanique à au moins une des première et deuxième surfaces principales de la bande d'onglet de support.

11. Procédé selon la revendication 10, dans lequel le ruban de liaison comprend une couche d'adhésif sensible à la pression faisant face à la bande d'onglet de base et à la bande d'onglet de support, l'adhésif sensible à la pression étant compatible avec la technique de soudage utilisée pour fixer le ruban de liaison à l'onglet de base et à l'onglet de support.

12. Procédé selon l'une quelconque des revendications 10 et 11, dans lequel au moins une partie de la bande d'onglet de base comprend une matière en feuille élastique.

13. Procédé selon l'une quelconque des revendications 10 à 12, dans lequel la bande d'onglet de support est inélastique.

14. Procédé selon l'une quelconque des revendications 10 à 13, dans lequel le ruban de liaison est inélastique.

15. Procédé selon l'une quelconque des revendications 10 à 14, dans lequel la fixation de la bande de composant de fixation à la bande d'onglet de support comprend la fixation adhésive de la bande de composant de fixation à la bande d'onglet de support.

16. Procédé selon l'une quelconque des revendications 10 à 15, comprenant en outre la séparation de la bande composite en une pluralité de systèmes de fermeture distincts après la fixation de la bande de ruban de liaison à la bande d'onglet de base et à la bande d'onglet de support et la fixation de la bande de composant de fixation à la bande d'onglet de support, chaque système de fermeture distinct de la pluralité de systèmes de fermeture distincts comprend un onglet de base, un onglet de support, un ruban de liaison et un composant de fixation de la même largeur.
